# EUROPEAN PATENT APPLICATION

(11) **EP 2 639 577 A1**
(43) Date of publication of application: **18.09.2013**
(21) Application number: 12159974.0
(22) Date of filing: 16.03.2012
(51) Int. Cl.: G01N 27/407

(54) **Electrochemical gas sensor comprising an anion-exchange membrane**

(71) Applicant: SolviCore GmbH & Co KG, 63457 Hanau-Wolfgang (DE)
(72) Inventor: Dziallas, Holger, D-63538 Großkrotzenburg (DE); Byrknes, Jan, D-60486 Frankfurt am Main (DE); Eickes, Christian, D-6043 Frankfurt am Main (DE); Ghielmi, Alessandro, D-60325 Frankfurt am Main (DE)
(74) Representative: Starz, Karl Anton

(57) **Abstract**

The present invention is directed to electrochemical gas sensors for the detection of combustible, flammable or toxic gases and to catalyst-coated membranes (CCMs) used therein. The gas sensor comprises at least one solid anion exchange membrane (AEM), a sensing electrode and a counter electrode. The sensing electrode comprises catalytically active material and anionic ionomer material, the weight ratio between the catalyst material and the anionic ionomer material in the sensing electrode is in the range of 3/1 to 99/1, preferably in the range of 4/1 to 30/1.

Due to the use of anion exchange ionomer materials, the sensor can be made less expensive and suitable for high volume production. When applied for the detection of CO, the sensor shows good CO selectivity S(CO/H₂) in the presence of hydrogen.

## Description

### Description

The present invention is directed to an electrochemical gas sensor used for the detection of combustible, flammable or toxic gases such as CO (carbon monoxide), alcohol vapors, NOₓ (nitric oxides) and others. The gas sensor of the present invention comprises at least one solid anion-exchange membrane (AEM). Preferably, the gas sensor of the present invention is used for the detection of carbon monoxide (CO) at ambient temperatures in the presence of hydrogen gas. Further, suitable catalyst-coated membranes (CCMs) for use in such electrochemical gas sensors are disclosed.

### Background of the invention

Generally, a gas sensor (or gas detector) is a device which detects the presence of various gases within an area, usually as part of a safety system. This type of equipment is used to detect a gas leak and is typically interfaced with a control system so that a process can be automatically shut down. A gas sensor can also sound an alarm to operators in the area where the leak is occurring, giving them the opportunity to leave the area. This type of device is important because there are many gases that can be harmful to organic life, such as humans or animals.

Gas sensors can be used to detect combustible, flammable and toxic gases, and oxygen depletion. This type of device is used widely in industry and can be found in a variety of locations such as on oil rigs to monitor manufacturing processes and emerging technologies. They may also be used in firefighting. Gas sensors are usually battery operated and work in broad temperature ranges, preferably at ambient temperatures. They transmit warnings via a series of audible and visible impulses such as alarms and flashing lights, when dangerous levels of gas vapors are detected. As sensors measure a gas concentration, the sensor responds to a calibration gas, which serves as the reference point or scale. As a sensor's detection exceeds a preset alarm level, the alarm or impulse will be activated. As units, gas detectors are produced as portable or stationary devices.

There are three main types of electrochemical gas sensors; amperometric, potentiometric and conductometric sensors.

In the amperometric sensor type, a current signal is created when the analyte gas reacts in an electrochemical reaction in the electrode. In the potentiometric sensor type the electrode potential changes depending on the concentration of the analyte gas present. In the conductometric sensor type it is the conductivity of the electrolyte that changes depending on the concentration of the analyte gas. In an ideal sensor, the signal is proportional to the concentration of the analyte gas and exhibits low sensitivity to other gases as well as low sensitivity to changes in temperature and humidity.

An electrochemical gas sensor comprises at least a sensing electrode and a counter electrode. At the sensing electrode, the analyte gas interacts with the electrode. As an example, in the case of an amperometric proton-exchange membrane (PEM)-type sensor designed to detect CO, an electrochemical oxidation of CO will take place at the sensing electrode in the presence of moisture, according to the following reaction (in an acid electrolyte):

CO + HzO → CO₂ + 2 H⁺ + 2 e-

On the other hand, in the counter electrode there is a water formation reaction by combining protons, electrons and oxygen in a reduction reaction of oxygen (in an acid electrolyte):

2 H⁺ + 2 e- + 1/2 O₂ → H₂O

The resulting current (electron flow) will be measured as the signal, eventually after amplification.

Generally it is known that gas sensors can be made based on a technology analogous to PEM fuel cell technology using proton-exchange polymer type electrolytes such as Nafion19 ionomers. Electrochemical gas sensors for the detection of CO and other gases are known in the prior art. As an example, US 5,650,054 and US 5,573,648 disclose gas sensors based on proton-conductive membranes.

Due to their corrosive, acidic environment, gas sensors based on proton-conducting membranes need expensive stainless steel housings and carbon-based gas diffusion layers to overcome the corrosion problems. Therefore, these gas sensors are expensive and less suitable for consumer applications in households and residential homes. Thus there is a need for sensor systems which are less expensive and suitable for high volume applications.

US 2006/0096871 is directed to a carbon dioxide monitoring sensor using a anion exchange membrane in combination with a metal-oxide sensing electrode.

The present invention provides improved electrochemical gas sensors using anion-exchange membranes (AEM) and anion-exchange ionomer materials.

Basically, the combination of solid anion-exchange membranes with electrodes containing anionic ionomer for fuel cell application is known in the prior art (ref to J.R. Varcoe and R.T.C. Slade, Fuel Cells 2005, 5(2), 187-200). In various publications, among other details, values of the catalyst/ionomer weight ratio higher than 1.5/1, up to 10/1 and even higher have been reported for the electrodes. Reference is made to the articles of H. Yanagi and K. Fukuta, ECS Transactions 16(2), 257-262 (2008) and of M. Mamlouk et al., Int. J. Hydrogen Energy, 2011, 36 (12), 7191-7198. In the recent patent literature, broad catalyst/ionomer weight ratios, ranging from 4/1 (in WO 2011/043758A1) to 10/1 (in US 2010/0216052A1) are disclosed. A similar broad range of catalyst/ionomer weight ratios for electrodes is also described in the prior art related to PEM technology based on proton-conductive ionomer materials.

### Summary of the invention

It is an object of the present invention to provide an improved electrochemical gas sensor comprising an anion-exchange membrane (AEM). The AEM-containing gas sensor is suitable for use in sensors for the detection of various gases such as carbon monoxide (CO), alcohol vapors, nitric oxides (NOₓ) and other toxic gases, preferably for the detection of CO in the presence of hydrogen (H₂). As an important feature, the sensor provides a high selectivity to CO in the presence of hydrogen and a rapid response time.

It is a further object of the present invention to provide suitable catalyst-coated membranes (CCMs) for use in electrochemical gas sensors, which comprise an anion-exchange membrane (AEM).

While extensive work is ongoing since decades in the fields of catalyst-coated membranes (CCMs) and membrane-electrode assemblies (MEAs) for PEM fuel cells, further work is necessary to improve the related CCM and MEA products based on anion exchange membranes (AEMs). Generally, the performance of the CCM products of the prior art is not sufficient for commercial use. This applies specifically to catalyst-coated membranes for use in gas sensor applications. To our knowledge, no gas sensor elements based on anionic ionomer CCMs have been described as of this date. This may be mainly due to the impossibility to obtain a sensor working with sufficient signal and selectivity for any practical use.

### Detailed description of the invention

In the following, the electrochemical gas sensor of the present invention and its components are described in further detail.

**Ficiure 1** shows a schematic drawing of the gas sensor of the present invention in the potentiometric and amperometric mode. Hydroxide ions (OH-) are conducted through an anion exchange membrane (AEM) located between the sensing and the counter electrodes. In the CO sensor of the present invention, the electrochemical oxidation of CO will take place at the sensing electrode (1) according to the following equation:

2 CO + 4 OH- → 2 CO₂ + 2 H₂O + 4 e-

In the counter electrode (2), the reduction of oxygen occurs in an alkaline electrolyte environment:

O₂ + 2 H₂O + 4 e- → 4 OH-

The exchange of the hydroxyl-anions (OH- ions) takes place within the solid anion exchange membrane (3); the electrons are traveling from the sensing electrode (1) to the counter electrode (2) under a certain potential difference, thus creating a current. The measurement of the signal output can be made in two different versions. In the potentiometric gas sensor version, a voltmeter V measures the potential difference between the electrical leads connected with the sensor electrodes (shown in solid lines); in the amperometric sensor embodiment (shown in dotted lines), an ammeter A in combination with a resistor element R_{L} provides the measurement of the corresponding signal current.

The present invention is directed to an electrochemical gas sensor for the detection of combustible, flammable or toxic gases, such as, e.g., carbon monoxide (CO), comprising at least one ionomer membrane (3), a sensing electrode (1) and a counter electrode (2), wherein the ionomer membrane is a solid anion exchange membrane (AEM) and the sensing electrode (1) and the counter electrode (2) comprise catalytically active material and anionic ionomer material.

The sensing electrode (1) and/or the counter electrode (2) are attached to the at least one ionomer membrane (3) thus forming at least one catalyst-coated membrane (CCM). The weight ratio between the catalytically active material and the anionic ionomer material in the sensing electrode (1) is in the range of 3/1 to 99/1, preferably in the range of 4/1 to 30/1.

In a first embodiment A (shown in **Ficiure 2**), the present invention provides an electrochemical gas sensor containing a catalyst-coated membrane (CCM), which comprises a solid anion-exchange membrane (3) and two electrode layers (1, 2) comprising electrocatalyst material and anionic ionomer. The two electrode layers are coated to the opposite sides of one ionomer membrane (3). Hereinafter, this embodiment is named "sensor element based on a 3-layer CCM".

In a second embodiment B, the invention provides an electrochemical gas sensor containing two catalyst-coated membranes (CCMs), each comprising a solid anion-exchange membrane (3, 3') coated with only one electrode layer (1 or 2) on one side, the electrode layer comprising electrocatalyst material and anionic ionomer. The reverse side of the membrane remains non-coated. Hereinafter, this embodiment 2 is named "sensor element based on a 2-layer CCM". In this second embodiment B, the non-coated membrane sides of the two 2-layer CCMs are facing each other (ref to **Ficiure 3a**). When the two membranes are in direct contact with each other, the sensor element is conceptually equivalent to a sensor element based on a 3-layer CCM; however, it comprises two anion exchange membranes, which may be identical or different from each other.

In a third embodiment C (ref to **Ficiure 3b**), the non-coated membrane sides of the two 2-layer CCMs are facing each other, however, they are separated by an ion conducting liquid or liquid electrolyte (5). This embodiment is also based on two 2-layer CCMs, thus it contains a first CCM comprising sensing electrode (1) on AEM (3) and a second CCM comprising counter electrode (2) on AEM (3'). The ion conducting electrolyte (5) is located between the non-coated areas of the anion exchange membranes (3, 3') .

### Anion exchange membranes and ionomer materials

In general, solid anion-exchange membranes comprise anion-exchange ionic polymers (anionic ionomers). As a rule, such anionic ionomers contain a main chain polymer having fixed positive charges (cationic groups) for the coordination of the negative mobile charges (anionic species, OH- ions). Some of these materials are based on quaternary ammonium (QA) ion-exchange functional groups (such as Morgane^{®} ADP from Solvay S.A. and Neosepta^{®} AHA from Tokuyama Soda). Other solid anion-exchange membranes are the grades A-201 and A-901 available from Tokuyama Soda. These membranes have a hydrocarbon main chain and QA groups for anion exchange (ref to H. Yanagi and K. Fukuta, cited above).

Anionic ionomers suitable for use in the present invention, both for the membrane and for use in the electrode can be selected from a variety of different types. In contrast to the fuel cell application, the environment for the gas sensor application in not very aggressive and often the gas sensor will be in the waiting mode until an event occurs where the gas to be detected is released to the environment. For sensors detecting e.g. poisonous gases such as CO, it most often happens that the sensor is never exposed to the poisonous gas for the whole life of the sensor. Besides, the sensor has to deliver a detectable signal but has no stringent requirements in terms of specific power output as required for fuel cells. Therefore, a broad class of anionic ionomers may be used for such application.

Suitable anionic ionomers are either hydrocarbon polymers or fluorinated polymers, bearing positively charged cationic groups such as ammonium, phosphonium, sulfonium, guanidinium or imidazolium groups. Quaternary ammonium (QA) groups are preferred.

Anionic ionomers may also be provided in the form of liquid compositions, namely in the form of solutions or dispersions. A suitable commercial ionomer solution product is available from Tokuyama Soda. This ionomer solution is named "AS-4"; it is a hydrocarbon type ionic polymer which contains quaternary ammonium (QA) groups (ref to ECS Transactions, 2008, 16 (2), 257-262, cited above). Other non-commercial types of anionic ionomer liquid compositions are e.g. the "SION1" developed by the University of Surrey (cf. J.R. Varcoe et al., ECS Transactions, 2008, 16 (2), 1819) and tris-(2,4,6-trimethoxyphenyl) phosphine based quaternary phosphonium polysulfone hydroxide (TPQPOH) from the Regents University of California (cf. WO 2011/043758A1).

Anionic ionomers useful for the sensor electrodes are preferably soluble or dispersible in a liquid medium, so that an ink can be obtained containing the electrocatalyst together with the ionomer which is then cast into an electrode using methods known in the art.

### The sensing electrode

Generally, the sensing electrode layer comprises an electrically conductive material which is catalytically active to facilitate the electrochemical reaction of the gas which needs to be detected. In the CO sensor of the present invention, this refers to the electrochemical oxidation of CO at the sensing electrode (1). The catalytically active material should be electrically conductive to provide for the flow of electrons across the electrode. The catalytically active material may be a precious metal, a base metal, a precious metal on a carbon support, a base metal on a carbon support, a precious metal on a conductive base metal support, a precious metal on a conductive metal oxide support or a base metal on a conductive metal oxide support.

Preferably, the catalytically active material comprises a precious metal selected from the group consisting of ruthenium (Ru), osmium (Os), rhodium (Rh), iridium (Ir), palladium (Pd), platinum (Pt), silver (Ag) and gold (Au) and mixtures and combinations thereof.

Further, the catalytically active material may be a base metal selected from the group consisting of vanadium (V), chromium (Cr), molybdenum (Mo), tungsten (W), manganese (Mn), iron (Fe), cobalt (Co), nickel (Ni), copper (Cu) and zinc (Zn) and mixtures and alloys thereof.

Optionally, the catalytically active material may be a precious metal admixed or alloyed with one or more base metals listed above, preferably it may be a precious metal alloyed or admixed with nickel (Ni), chromium (Cr), cobalt (Co) or copper (Cu).

In a preferred embodiment, the catalytically active material is a precious metal supported on a conductive support such as carbon black, graphite or conductive metal oxides such as, e.g., indium-tin oxide powders. Typical examples for suitable catalytically active conductive materials are electrocatalysts such as 40 wt.-% Pt/C, 60 wt.-% PtCo/C or 20 wt.-% PtNi/C. In the latter compositions, the Pt may be alloyed or coated with the base metal. Such catalyst materials are commercially available from different vendors. In a specific embodiment, the catalytically active material may be a precious metal supported on a base metal powder support.

Due to the alkaline environment provided by the anion-exchange ionomer, it is generally not necessary to use precious metals as catalysts for the electrochemical sensor of the present invention. Base metals selected from the group listed above; in particular nickel (Ni), chromium (Cr) cobalt (Co) or alloys thereof in the form of fine powders or nanoparticles may be employed in admixture with the anion-exchange ionomer.

Typically, the catalytically active material should be a finely divided, powder-type material with a high active surface area (BET-surface area) in the range of 10 to 150 m²/g, preferably in the range of 20 to 120 m²/g. The medium particle size of the catalytically active material should be in the range of 2 nm to 10.000 nm (10 µm).

### Sensor selectivity S

With good gas selectivity S, it is meant that the signal associated with one type of gas is significantly different than the signal associated with another type of gas at the same concentration, so that the sensor is more sensitive to the gas to be detected compared to other gases, thus avoiding false signals from the sensor. In reference to the present invention, for a sensor designed to detect CO as dangerous gas in the presence of hydrogen, it is desired that the sensor does not produce a significant signal when H₂ is present at similar levels in the environment.

Generally, the selectivity S between two gases A and B may be expressed as the ratio of the signals delivered by the sensor element in the presence of gas A and in the presence of gas B at the same concentration of A and B in a carrier gas (e.g., ambient air). As an example, for the amperometric type CO sensor two different measurements are conducted. In the first measurement, the current intensity signal (I in µA) in the presence of 1.000 ppm of CO in the carrier gas air is detected. In the second measurement, the current intensity signal (I in µA) resulting from 1.000 ppm hydrogen in the carrier gas air is determined. The gas sensor selectivity S for the detection of CO in the presence of hydrogen is given by the formula

S (CO/H₂) = I (CO@1000 ppm) / I (H₂@1000 ppm),

wherein I (in µA) is the current intensity detected by the sensor. As can be seen from **Table 1** in the Examples section, the gas sensor according to the present invention shows very good results. The selectivity S(CO/H₂) as determined by the ratio of the current intensity signals [I_{CO/}I_{H2]} is typically ≥ 3.

Finally, with the term good signal intensity it is meant that the sensor gives a good response, e.g. in terms of current or voltage, already at low levels of concentration of the gas to be detected.

### The catalyst/ionomer ratio

Hereinafter, the weight ratio between the catalytically active material and the anionic ionomer material in a sensor electrode is called "catalyst/ionomer ratio". It has been found by the present inventors that CO sensors with good signal intensity and high selectivity can be obtained by employing anionic ionomer materials. More specifically, it has been found, surprisingly, that good gas selectivity properties can be obtained by catalyst/ionomer weight ratios in the sensing electrode that are high enough, namely higher than 3/1. Good signal intensity and excellent selectivity properties can be obtained by increasing further the catalyst/ionomer weight ratio in the sensing electrode, namely to values higher than 6/1.

Surprisingly, it has been found that by increasing the values of the catalyst/ionomer weight ratio in the sensing electrode to values higher than 3/1, good values of selectivity S can be achieved. This result is surprising, since better selectivities would be expected at increasing ionomer amounts (i.e. with decreasing catalyst/ionomer weight ratio). In fact, at increasing ionomer amounts it should be expected that the ionomer completely covers the catalyst, preferentially allowing faster transport to the catalyst surface of those gases having a higher permeability through the ionomer (e.g. small or soluble molecules), thus giving selectivity properties to the electrode.

Contrary to this expectation, the inventors found that high ionomer amounts in the sensing electrode lead to sensors with no gas selectivity. Sensors containing sensing electrodes with catalyst/ionomer weight ratios of 2/1 deliver insufficient signal intensities and no selectivity (Ref to Comparative Example 1, CE1). It should be noted that ratios of 2/1 are typical in PEM and AEM fuel cell application.

For the present invention, in order to obtain working gas sensors, it is necessary that the catalyst/ionomer weight ratio in the sensing electrode is higher than 3/1, preferably higher than 4/1 and particularly preferred higher than 6/1. The catalyst/ionomer weight ratio in the sensing electrode should be lower than 99/1 to allow that the catalyst is contacted and bound by the ionomer and the electrode can be easily fabricated and preserves sufficient ionic conductivity. Preferably, the catalyst/ionomer weight ratio should be lower than 30/1 and particularly preferred, the catalyst/ionomer weight ratio should be lower than 20/1.

In summary, it was found by the present inventors that the catalyst/ionomer weight ratio in the sensing electrode should be in the range of 3/1 to 99/1, preferably in the range of 4/1 to 30/1 and particularly preferred in the range of 6/1 to 20/1.

While the catalyst/ionomer weight ratio can be directly measured, the catalyst/ionomer ratio in an electrode layer may also be expressed in terms of volumetric ratio (volume of catalyst/volume of ionomer). It is sometimes claimed in the technical literature that the properties of the electrode are determined by the relative volume of the components in the electrode rather than by the masses as such. The catalyst/ionomer volumetric ratio can be calculated from the weight ratio by knowing the densities of the different components. Assuming typical density values for a catalyst and for an anionic ionomer, the preferred intervals above can be expressed in terms of volumetric ratio intervals. The catalyst/ionomer ratio (volumetric) in the sensing electrode is found to be in the range of 0.7/1 and 33/1, preferably in the range of 1/1 and 10/1, more preferably in the range of 1.5/1 and 5/1.

### The counter electrode

Generally, in the counter electrode, the electrochemical reduction of oxygen occurs in an alkaline electrolyte environment as previously described. As outlined in the section describing the sensing electrode, the catalytically active material should be electrically conductive to provide for the flow of electrons across the electrode. The catalytically active material may be, independently from the composition of the sensing electrode, a precious metal, a base metal, a precious metal on a carbon support, a base metal on a carbon support, a precious metal on a conductive base metal support, a precious metal on a conductive metal oxide support or a base metal on a conductive metal oxide support. The definition of precious metals and base metals apply accordingly.

The composition of the counter electrode is not particularly limited and may be the same or different from that of the sensing electrode. For the sake of clarity, catalyst/ionomer weight ratios outside the range specified above for the sensing electrode can be employed. Preferably, for simplicity of production of the sensor, the same electrode composition may be used for the sensing and the counter electrode, so that no mistake can be made when assembling the CCM(s) in the sensor.

In the sensor system, the counter electrode (2) is typically in contact with the water vapor arising from a water solution reservoir via a hydrophobic micro-porous membrane, e.g. an expanded PTFE membrane.

### Electrode fabrication

The sensing and/or counter electrodes may be typically fabricated directly in contact with the membrane or prepared onto a supporting film and then transferred by pressure and heat to the surface of the membrane (decal process). In the decal process the electrode is fabricated on a supporting film by casting a catalyst ink using techniques known in the art and drying the wet layer at temperatures typically above 40°C and normally not higher than 200°C. Drying temperatures lower than 150°C are normally preferred to prevent thermal degradation of the anionic ionomer.

The transfer of the dry electrode to the surface of the anion-exchange membrane is then performed by placing the electrode layer on the supporting film against the surface of the membrane and applying pressure and heat on the package, for example by using a press or a lamination machine with heated rolls or heated belts. Typical transfer pressures are between 3 and 30 bars and typical transfer temperatures are between 50 and 200°C, preferably below 150°C. Pressure, temperature and time for optimal electrode transfer and optimal performance depend in general on the composition of the catalyst layer, the membrane type and the equipment employed, and need to be determined on a case to case basis.

### Sensor construction

The sensor of the invention may be constructed in a variety of versions. In general, the sensing electrode will be exposed to the atmosphere but should be protected from ambient dust by a particle filter. Also, a gas permeable membrane may be used to limit the gas access on the side of the sensing electrode in order to have the electrode working under mass transport control rather than under kinetic control. This feature remedies an eventual change in time of the activity of the electrode. The sensor may include a water or water solution reservoir (e.g. water and anti-freezing agent) to keep the catalyst coated membrane(s) at a constant relative humidity (RH) thus providing a higher stability signal at varying environmental RH conditions. A gel, e.g. silica gel, may be added to the water reservoir to enhance the durability of the sensor at high temperatures.

In the embodiment shown in **Ficiure 3b**, where two 2-layer CCMs are separated by a liquid electrolyte, proper confinement or gasketing must be provided to ensure that the liquid electrolyte does not spill out of the casing. As electrolyte, a liquid medium (preferably water) containing any soluble compound or salt conferring anion-exchange characteristics to the solution can be employed, provided that this is stable in time and not chemically aggressive to the membranes and to the casing. In the simplest case, alkaline metal hydroxide solutions in water, such as NaOH and KOH can be employed.

The sensor may comprise, besides a sensing and a counter electrode, also a reference electrode. This will in general be composed of materials similar in nature to the sensing and counter electrodes, i.e. comprising an electrocatalyst and an anion-exchange ionomer. The sensor may additionally comprise a further pair of electrodes or even an additional CCM, the scope of which is to pump (electrochemically) the analyte gas out of the counter electrode. This concept is exemplified in US 5,650,054 with reference to the use of solid proton-exchange materials in the sensor. The feature of gas pumping provides a higher accuracy in the detection of the analyte gas at the sensing electrode. The sensor must in this case be endowed with an additional DC power supply connected to the pumping pair of electrodes.

The casing of the sensor may be fabricated out of conductive material, e.g. metal, in which case it can be connected to one of the electrodes, or can be fabricated of non-conductive material, e.g. plastic, in which case the wires must be connected directly to both electrodes. Also the circuitry connected to the electrodes can be diverse. As stated above, an ammeter will be integrated to measure the signal current in the amperometric mode while a voltmeter will be inserted in the circuit in the potentiometric mode. A DC power source may also be inserted in the circuit as a driving force for analyte gas oxidation, the signal current in the presence of the analyte gas being determined in this case as the difference to the background current generated by the DC source in the absence of the analyte gas. The DC source is in general necessary when the resistance of the electrodes and/or electrolyte is high. This is typically not the case for the present invention, where a thin membrane is used as an electrolyte and the electrodes comprise a highly electronically conductive material and an anion-exchange polymer. If not necessary to generate a measurable signal, the presence of the DC power source is preferably avoided since it enhances the ageing of the electrodes.

The following examples shall further describe the invention without limiting or narrowing its scope.

### Example 1

A CCM for CO-sensing is fabricated using the following materials: Electrocatalyst (60 wt.-% Pt on carbon black support; Elyst™ AC 60, Umicore AG & Co KG, Hanau, Germany); anionic ionomer solution (5 wt.-% ionomer in 1-propanol (type AS-4, Tokuyama Corporation, Tokyo, Japan) and anionic ionomer membrane (type A-201, Tokuyama Corporation, Tokyo, Japan). First, the concentration of ionomer in the AS-4 solution is increased from 5 wt.-% as delivered to 9 wt.-% by controlled evaporation in a rotary evaporator. 44.0 g of this ionomer solution with increased ionomer content is mixed with 15.9 g of electrocatalyst and 40.0 g of deionized water for 2 hours under vigorous stirring at 2°C. The catalyst dispersion prepared according to this procedure is applied to a fluorinated substrate film by a doctor blade method using an automatic film applicator and dried in a belt-type furnace at a peak temperature of 50°C in 8 minutes.

The resulting "decal-type" electrode layers are hot pressed onto both sides of the A-201 anionic membrane at 120°C for 3 minutes with a subsequent cooling step at ∼15°C for 2 minutes. The catalyst/ionomer ratio of the electrodes is 4/1 by weight. The resulting 3-layer CCM is assembled into an amperometric sensor for detecting poisonous CO gas and hydrogen gas. The CO and hydrogen signals of the sensor (given in µA) and the CO selectivity versus H₂, specified by S(CO/H₂), are reported in **Table 1**. The sensor element yields a weak signal but shows a good CO selectivity in the presence of hydrogen.

### Example 2

A further sensor element is fabricated using the materials as described in Example 1. As in Example 1, the concentration of ionomer in the AS-4 solution is increased from 5 wt.-% as delivered to 9 wt.-% by controlled evaporation in a rotary evaporator. 26.6 g of this ionomer solution with increased ionomer content is mixed with 14.4 g of the electrocatalyst and 32 g of deionized water and 7.0 g of 1-propanol (Analytical grade, Merck) for 2 hours under vigorous stirring at 2°C. The catalyst dispersion is applied to a fluorinated substrate film by a doctor blade method using an automatic film applicator and dried in a belt-type furnace at a maximum temperature of 50°C in 8 minutes. The resulting decal type electrodes are hot pressed onto both sides of the anionic membrane at 120°C for 3 minutes with a subsequent cooling step at ∼15°C for 2 minutes. The catalyst/ionomer ratio of the electrodes is 6/1 by weight.

The resulting 3-layer CCM is assembled into an amperometric sensor for detecting poisonous CO gas and hydrogen gas. The CO and hydrogen signals of the sensor (given in µA) and the CO selectivity versus H₂, specified by S(CO/H₂), are reported in **Table 1**. The sensor yields a weak signal but shows a good selectivity S.

### Example 3

A further version of the sensor element as described in Example 1 is-prepared in the following example. The materials as described in Example 1 are used. The concentration of ionomer in the AS-4 solution is increased from 5 wt.-% as delivered to 9 wt.-% by controlled evaporation in a rotary evaporator as described in the previous examples.

22.2 g of this ionomer solution with increased ionomer content is mixed with 16.1 g of the electrocatalyst, 32.0 g of deionized water and 9.8 g of 1-propanol (analytical grade, Merck) for 2 hours under vigorous stirring at 2°C. The electrocatalyst dispersion is applied to a fluorinated substrate film by a doctor blade method using an automatic film applicator and dried in a belt-type furnace at a peak temperature of 50°C in 8 minutes. The resulting decal type electrodes are hot pressed onto both sides of the anionic membrane at 120°C for 3 minutes with a subsequent cooling step at ∼15°C for 2 minutes. The catalyst/ionomer ratio of the electrodes is 8/1 by weight.

The resulting 3-layer CCM is assembled into an amperometric sensor for detecting poisonous CO gas and hydrogen gas. The CO and hydrogen signals of the sensor (given in µA) and the CO selectivity versus H₂, specified by S(CO/H₂), are reported in **Table 1**. The sensor element yields a strong signal and shows a high selectivity of S = 4.42.

### Example 4

Example 3 is duplicated, except that the catalyst dispersion prepared in Example 2 is used to fabricate the counter electrode. The CCM is therefore asymmetric in this case, i.e., with different catalyst/ionomer ratios in the sensing electrode (= 8/1) and in the counter electrode (= 6/1).

The resulting sensor characteristics (signal strength and selectivity) are very similar to those obtained with the CCM of Example 3.

### Example 5

Example 3 is duplicated, except that a 50 wt.-% Pt on carbon black support is used as catalyst. The resulting sensor characteristics (signal strength and selectivity) are very similar to those obtained with the CCM of Example 3.

### ComDarative ExamDle 1 (CE1)

A gas sensor element is fabricated using the materials as described in Examples 1-3; the concentration of the AS-4 ionomer solution is increased from 5 wt.-% as delivered to 9 wt.-% by controlled evaporation in a rotary evaporator as described.

68.0 g of this ionomer solution with increased ionomer content is mixed with 12.0 g of the electrocatalyst and 0.4 g of 1-propanol (Analytical grade, Merck) for 2 hours under vigorous stirring at 2°C. The catalyst dispersion is applied to a fluorinated substrate film by a doctor blade method using an automatic film applicator and dried in a through-type furnace at a maximum temperature of 50°C in 8 minutes. The resulting decal type electrodes are hot pressed onto both sides of the anionic membrane at 120°C for 3 minutes with a subsequent cooling step at ∼15°C for 2 minutes. The catalyst/ionomer ratio of the electrodes is 2/1 by weight.

The resulting 3-layer CCM is assembled into an amperometric sensor for detecting poisonous CO gas and hydrogen gas. The CO and hydrogen signals of the sensor (given in µA) and the CO selectivity versus H₂, specified by S(CO/H₂), are reported in **Table 1***.* The sensor element gives a weak signal and shows no selectivity (i.e. selectivity S very close to 1). Thus, the gas sensor comprising this CCM is not suitable for practical applications.

### Electrochemical testing

In the examples given above, gas sensor elements are assembled with anionic ionomer-based CCMs (AEM) and tested for CO detection and for selectivity to hydrogen H₂ at ambient temperature. The current generated by the sensor element is measured (in the amperometric mode) in two different measurements with concentrations of CO and respectively H₂, each at concentrations of 1.000 ppm in air atmosphere.

For both measurements, the signal from the sensor is reported as the current I (in µA) generated by the sensor. The selectivity S(CO/H₂) is calculated as the ratio between the individual current signal intensity generated by the sensor at 1.000 ppm of CO vs. 1.000 ppm of H₂, respectively. Results are given in **Table 1.**

As can be seen from the table, the gas sensor according to the present invention shows very good selectivity values ranging from 3.11 to 4.5; the selectivity S(CO/H₂) as determined by the ratio of the current intensity signals [I_{CP}/I_{H2}] is typically≥ 3.

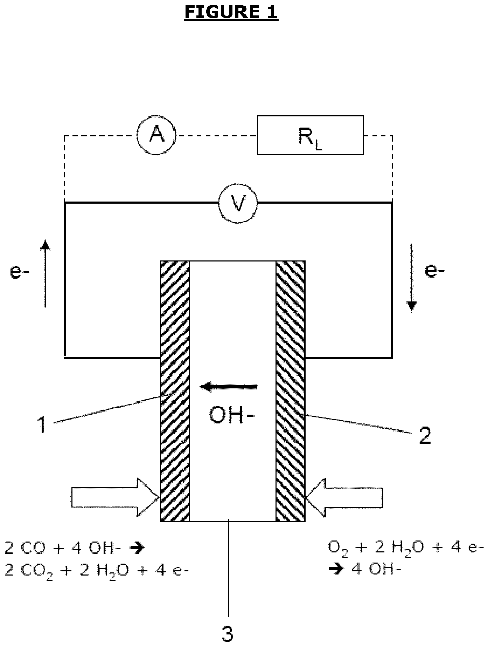

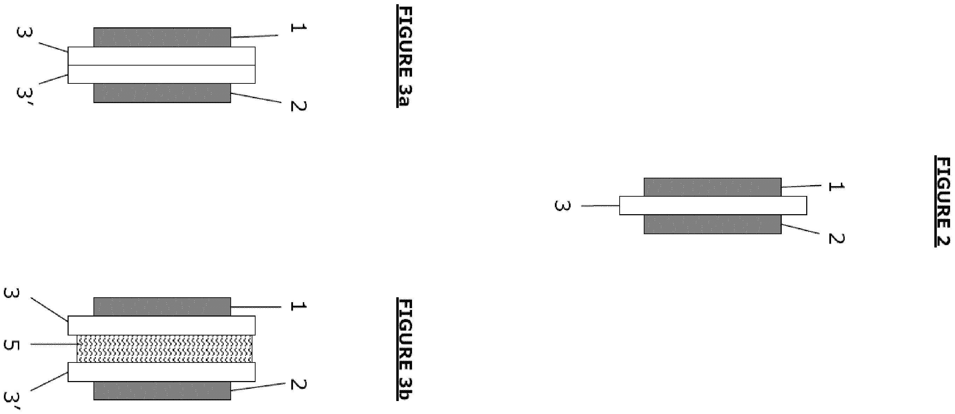

## Claims

1. Electrochemical gas sensor for the detection of combustible, flammable or toxic gases, comprising at least one ionomer membrane (3), a sensing electrode (1) and a counter electrode (2), wherein the ionomer membrane (3) is a solid anion exchange membrane (AEM) and the sensing electrode (1) and the counter electrode (2) comprise catalytically active material and anionic ionomer material.

2. The sensor according to claim 1, having a high selectivity S(CO/H₂) for the detection of carbon monoxide (CO) in the presence of hydrogen (H₂).

3. The sensor according to claim 1 or 2, wherein the sensing electrode (1) and/or the counter electrode (2) are attached to the at least one ionomer membrane (3) thus forming at least one catalyst-coated membrane (CCM).

4. The sensor according to any one of claim 1 to 3, wherein the weight ratio between the catalytically active material and the anionic ionomer material in the sensing electrode (1) is in the range of 3/1 to 99/1, preferably in the range of 4/1 to 30/1.

5. The sensor according to any one of claims 2 to 4, wherein the selectivity S(CO/H₂) for the detection of carbon monoxide (CO) in the presence of hydrogen (H₂) is ≥ 3 (as determined by the ratio of the current intensity signals [I_{CO}/I_{H2}]).

6. The sensor according to any one of claims 1 to 5, wherein the catalytically active material in the sensing electrode (1) and in the counter electrode (2) is electrically conductive.

7. The sensor according to any one of claims 1 to 6, wherein the catalytically active material in the sensing electrode (1) and/or in the counter electrode (2) is a precious metal selected from the group of ruthenium (Ru), osmium (Os), rhodium (Rh), iridium (Ir), palladium (Pd), platinum (Pt), silver (Ag) and gold (Au) and mixtures and combinations thereof.

8. The sensor according to any one of claims 1 to 6, wherein the catalytically active material in the sensing electrode (1) and/or in the counter electrode (2) is a base metal selected from the group of vanadium (V), chromium (Cr), molybdenum (Mo), tungsten (W), manganese (Mn), iron (Fe), cobalt (Co), nickel (Ni), copper (Cu) and zinc (Zn) and mixtures and alloys thereof.

9. The sensor according to any one of claims 1 to 6, wherein the catalytically active material in the sensing electrode (1) and/or in the counter electrode (2) is a mixture or an alloy of a precious metal with a base metal.

10. The sensor according to any one of claims 1 to 6, wherein the catalytically active material in the sensing electrode (1) and/or in the counter electrode (2) is a precious metal or a base metal or an alloy of a precious metal with a base metal supported on conductive carbon black or on a conductive metal oxide support.

11. The sensor according to any one of claims 1 to 10, wherein the solid anion exchange membrane is based on a hydrocarbon polymer containing quaternary ammonium (QA) ion-exchange functional groups.

12. The sensor according to any one of claims 1 to 11, wherein the anionic ionomer material in the electrodes is based on a hydrocarbon polymer containing quaternary ammonium (QA) ion-exchange functional groups.

13. The sensor according to any one of claims 1 to 12, wherein the sensing electrode (1) and the counter electrode (2) are attached to the opposite sides of the solid anion-exchange membrane (3).

14. The sensor according to any one of claims 1 to 12, wherein the sensing electrode (1) is attached directly to a first solid anion-exchange membrane (3) and the counter electrode is attached to a second solid anion-exchange membrane (3') and wherein the two catalyst-coated membranes (1, 3) and (2, 3') are arranged in such a way that the two solid anion-exchange membranes (3, 3') are facing each other.

15. The sensor according to claim 14, wherein the first and the second solid anion-exchange membranes (3, 3') are placed in direct contact with each other and optionally are bonded together.

16. The sensor according to claim 14, wherein an ionic conducting liquid (5) is arranged between the first and the second solid anion-exchange membrane (3, 3').

17. The sensor according to any one of claims 1 to 16, further comprising a reference electrode, a water reservoir, housing, electrical wiring and optionally a gasketing material.

18. Catalyst-coated ionomer membrane (CCM) in an electrochemical gas sensor, comprising a solid anion exchange membrane (AEM) and a sensing electrode containing catalytically active material and anionic ionomer material, wherein the weight ratio between the catalytically active material and the anionic ionomer material in the sensing electrode is in the range of 3/1 to 99/1, preferably in the range of 4/1 to 30/1.

19. Catalyst-coated ionomer membrane according to claim 18, wherein the selectivity S(CO/H₂) for the detection of carbon monoxide (CO) in the presence of hydrogen (H₂) is ≥ 3 (as determined by the ratio of the current intensity signals [I_{CO}/I_{H2}]).

20. Catalyst-coated ionomer membrane according to claim 18 or 19, wherein the solid anion exchange membrane is based on a hydrocarbon polymer containing quaternary ammonium (QA) ion-exchange functional groups.
